# EUROPEAN PATENT SPECIFICATION

(11) **EP 1 079 800 B1**
(45) Date of publication and mention of the grant of the patent: **25.09.2002**
(21) Application number: 99923186.3
(22) Date of filing: 19.05.1999
(51) Int. Cl.: A61K 7/50, A61K 7/00, A61K 7/06, A61K 7/48

(54) **CLEANING COMPOSITION**
REINIGUNGSZUSAMMENSETZUNG
COMPOSITION DE NETTOYAGE

(30) Priority: 28.05.1998 US 86427
(43) Date of publication of application: 07.03.2001
(73) Proprietor: Colgate-Palmolive Company, New York, N.Y. 10022 (US)
(72) Inventor: HARMALKER, Subhash, Somerset, NJ 08873 (US)
(74) Representative: Prins, Adrianus Willem
(86) International application number: US9910941
(87) International publication number: WO99060996

(56) References cited:
- EP-A- 0 463 780
- EP-A- 0 738 509
- WO-A-97/32568

## Description

### BACKGROUND OF THE INVENTION

There exists a continuing need for preparing cleansing products which perform the proper cleansing, are mild to the skin, condition the skin and are aesthetically pleasing as well. Recently, there have been a number of "2 in 1" products which have attempted to provide these functions in one product. However, these products have a number of disadvantages. For example, the products are marketed as a heavy emulsion which has a tendency to separate in time and is not aesthetically pleasing. Additionally, such systems may have particles which are not stable at long term thereby liberating their contents or are systems wherein the emollient carrying materials are not stably suspended over time and/or the aqueous phase becomes turbid and changes color over time.

Systems have been developed which have made demonstrative steps in solving these problems. Chief among these is USP 5,681,801 (801). This patent discloses a clear aqueous cleansing system having stably suspended therein particles bearing water insoluble or essentially insoluble skin or hair conditioning agents. The clarity and stable suspension of the particles is achieved through the use of a xanthan gum having certain properties.

A new composition has been discovered which provides an aqueous cleansing composition, preferably clear, having stably suspended therein particles bearing water insoluble or essentially water insoluble skin or hair conditioning agents. The clarity and stable suspension is achieved with a mixture of a certain xanthan gum and a certain guar gum. This mixture not only stably suspends the particles, with no loss in clarity, but also provides greater clarity and concomitantly increased viscosity as opposed to using xanthan gum alone. A further advantage is that the tendency of the prior art '801 composition containing only xanthan gum to squirt from its container when subjected to pressure is substantially reduced or essentially eliminated when the xanthan gum is combined with the guar gum of this invention. The economics of the new composition is very similar to the '801 composition.

Various other combinations of xanthan gum with a gelation, viscosity enhancing component did not bring about the set of properties which the invention mixture possesses, particularly the enhanced clarity coupled with the increased viscosity. Examples of these potential agents which did not bring about the desired properties when combined with xanthan gum include other guar gums, alginate gum(s), (Ketone NV), an acrylate C₁₀₋₃₀ alkyl acrylate crosspolymer (Carbopol ETD 2020), hydroxyalkyl (propyl) methyl cellulose (Methocel J12MS from Dow) and a standard thickener such as lauramide diethanolamide.

### SUMMARY OF THE INVENTION

In accordance with the invention, there is a composition comprising a stable, clear aqueous cleansing phase having stably dispersed and suspended therein particles bearing conditioning agents comprising:
a. a high foaming anionic surfactant,
b. an amphoteric surfactant in quantities from zero wt % of the composition to a quantity wherein clarity of the aqueous phase is maintained,
c. an effective amount of a viscoelastic enhancing, suspending material comprised of a xanthan gum said xanthan gum having an initial transparency of at least 85% and a guar gum, said guar gum having an initial transparency of at least 85%,
d. insoluble particles stably suspended in the aqueous phase, bearing an oily, water insoluble or essentially water insoluble skin or hair conditioning agent.

### DETAILED DESCRIPTION OF THE INVENTION

Any high foaming anionic surfactant can be employed in the composition. Examples of such surfactants include but are not limited to long chain alkyl (8-20 carbon atom, preferably 10-18) materials such as long chain alkyl sulfates, long chain alkyl sulfonates, long chain alkyl phosphates, long chain alkyl ether sulfates, long chain alkyl alpha olefin sulfonates, long chain alkyl taurates, long chain alkyl isethionates (SCI), long chain alkyl glyceryl ether sulfonates (AGES), sulfosuccinates and the like. Those anionic surfactants can be alkoxylated, preferably ethoxylated, or not. The preferred anionic surfactants are the high foaming sulfates and sulfonates, particularly those which are ethoxylated such as sodium laureth (2 ethoxy) sulfate. All these materials are highly water soluble as the sodium, potassium, alkyl and ammonium or alkanol ammonium containing salt form and provide high foaming cleansing power. Since mildness is clearly a desirable attribute, it is preferred to have little or no anionic surfactant which has a high irritation potential. For example, fatty acid carboxylate soaps are present in limited quantity, for example less than about 2 wt. % of the composition, or not present at all. When using surfactants such as sulfates or sulfonates, preferably ethoxylated, the composition generally has less than about 3 wt. %, more preferably no presence of an additional anionic surfactant.

The quantity of the anionic surfactant is not unduly significant; however, minimum quantities of at least about 2 wt. % of the composition should be employed. Very little benefit above about 25 wt. % of the composition is generally gained. Generally, a range of about 5 to about 20 wt. %, preferably about 7 to about 13 wt. % of the composition can be employed. When appropriate, mixtures of anionic surfactants can be employed.

The second component of the cleansing phase is an amphoteric surfactant although it can be omitted completely if desired. These surfactants are generally known for their high surfactant activity, lather forming and mildness. Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyaminopropionate, sodium 3-dodecylaminopropane sulfonate, N-alkyltaurines, such as the one prepared by reacting dodecylamine with sodium isethionate according to the teaching of U.S.P. 2,658,072, N-higher alkyl aspartic acids, such as those produced according to the teaching of U.S.P. 2,438,091, and the products sold under the trade name "Miranol" and described in U.S.P. 2,528,278. Other amphoterics such as betaines are also useful in the present composition.

Examples of betaines useful herein include the high alkyl betaines such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl) carboxy methyl betaine, stearyl bix-(2-hydroxypropyl) carboxymethyl betaine, oleyl dimethyl gamma-carboxypropyl betaine, lauryl bix-(2-hydro-xypropyl) alpha-carboxyethyl betaine, etc. The sulfobetaines may be represented by coco dimethyl sulfopropyl betaine, stearyl dimethyl sulfopropyl betaine, amido betaines amidosulfobetaines, and the like. The betaines are generally preferred with the long chain alkyl group particularly coco, most preferred. Amido groups therein are also preferred. The most preferred betaines are the cocoamidopropyl or ethyl betaines.

The quantity of amphoteric agent if present, is not unduly significant but should be at least about 0.2 wt. % of the composition, preferably at least about 0.5 wt. %. However, the clarity of the aqueous phase should be maintained. The quantity of amphoteric reagent can affect the clarity. For example, with cocoamidopropylbetaine, a level not exceeding about 2 wt. % can be employed. Generally, there is not more than about 10 wt. %, assuming that clarity is maintained, preferably no more than about 7 wt. %.

A third component of the composition cleansing phase is not required but is preferred and is a nonionic, mild surfactant. Examples of such surfactants include EO-fatty alcohol, sorbitan and sorbitol esters, glucose ethers and alkylated polyglycosides amine oxides, alkanolamides (CDEA, CMEA) and the like. Preferred are alkanolamides and the alkylated polyglycosides (APG). The degrees of polymerization of the APG is preferably in a range of from about 1 to about 2, more preferably about 1.1 to about 1.8. The number of carbon atoms in the alkyl, preferably normal, range from about 8 to about 20 preferably about 10 to about 18. The alkanolamides have an alkyl group of the same length range as the APG alkyl. The APG can be alkoxylated, preferably ethoxylated, or left without ethoxylation. These materials are readily available from suppliers such as Emery, Henkel and Seppic.

The quantity of nonionic surfactants can vary substantially. Generally, a minimum of about 0.5 wt. % of the composition is employed. Above about 10 wt. % generally does not achieve substantial benefits and merely adds cost. A range of about 0.75 to about 7 wt. %, preferably about 1 to about 4 wt. % can be employed. The quantity of nonionic should not exceed that quantity which is able to maintain clarity of the composition.

From the nature of the invention, it is clear that any additional materials which may typically be present in aqueous compositions such as colorants, preservatives, chelating agents, fragrances, antibacterial agents and like can also be present in the composition as long as the clarity and stability of the system, as defined, can be maintained. The same criteria also applies to any additional "actives" such as surfactants.

The matching of these three components of the aqueous phase of the compositions brings about an excellent mix of properties for cleansing, such as high foam, long lasting controlled lather and a desirable mildness. Clarity is a significant characteristic of this phase and the clearness is maintained for lengthy periods of time. For example, clarity of the finished composition has been maintained for a period of at least two, preferably three months at a temperature of 43.3 ° C. This clarity is measured by turbidity. It is measured directly on the end product prior to addition of the particles and preferably any material (s) which may adversely affect the clarity. The clarity is evaluated visually or preferably by measuring on a turbidimeter in Nephelometric Turbidity Units (NTU). Acceptable turbidity is at most about 20 NTU. During stability testing, the aqueous phase without particles is measured visually or by the turbidimeter. The composition having the suspended particles is measured visually.

Within the clear aqueous phase are stably suspended particles which bear on and/or inside their surface conditioning agents. These conditioning agents include but are not limited to emollients, anti-oxidants, vitamins, oils, and any other oil like material which when applied to the skin or hair provides a conditioning effect. These particles have a density generally very close to that of water, 1 gm/cm³, for ease of suspension and stability. A preferred density range is about 0.92 to about 1.05 gm/cm³ more preferred, about 0.97 to about 1.02 gm/cm³.

The particle size should be such that it can bear the desired amount of conditioning agent and be readily physically degraded so as to release the conditioning agent. A further desired attribute of the particle is that it has optimal visual impact to the composition user. Generally, the minimum size of the particle is at least about 200 microns. Preferably, the particle size is at least about 400 microns, and even more preferably at least about 800 microns, and most preferably at least about 1000. The maximum size of the particle is not unduly significant but is generally no larger than about 2500, preferably no more than about 2000 or 1800 microns. The material comprising the particle should be compatible with water and the skin or hair. Generally, such materials can include gelatins, arabic gums, collagens, polypeptides from vegetable or animal origin, alginates, polyamides, glycosamino glycans, mucopolysaccharides, ethylcellulose and the like. Through coacervation, multicoating protein deposition, or reticulation technologies, microcapsules can be formed which enclose the oil like conditioning agents. The conditioning agents can be applied to the particle surfaces by impregnation.

Examples of "oily" materials which can be within the microcapsule are vitamins, provitamins, mineral oils, vegetable oils, emollients such as fatty esters and silicones, oil soluble vegetable extracts or animal extracts and the like. These oil bearing particles are available from Hallcrest Liquid Crystal Technology Ltd., a UK company having offices in Glenview, Illinois, U.S., and from LIPO Chemicals. Other materials which can be employed are oil impregnated particles available as Elesheres® from Laboratories Serobiologiques, France. Also, collagen spheres and glucose amino glycans "GAG" spheres from Coletics (France) called Collaspheres or Thalaspheres. The particles plus the conditioning agents borne thereon are generally at least about 0.02 wt. % of the composition, preferably at least about 0.03 wt. %. Generally, there is no specific maximum but because of various factors including cost, this is no more than about 3 wt. % of the composition, preferably not above about 2 or 1.5 wt. % of the composition. An effective amount of conditioning agent should be carried by the particles. Generally. at least about 40% of the loaded particle is conditioning agent, preferably at least about 55 wt. % of the particle. One of the loaded particles from Coletica of France is about 98 wt. % conditioning agent.

The combination of properties of the composition with respect to the clarity and the stable suspension of particles is brought about by the mixture of the xanthan gum and the guar gum. The xanthan gum is, in general, hydrophilic colloids made up of a polymer backbone of β-1, 4-linked D-glucose residues. A trisaccharide branch contains one glucuronic acid unit between two mannose units and is linked to every other glucose unit at the number 3 position. The xanthan gum has a light transmission of at least about 85% when measured as a 1 wt. % solution in distilled water using a UV spectrophotometer such as a Pye Unicam at 600nm. Distilled water is used as a 100% transmission standard. Preferred transmission is at least about 90%. This characteristic preferably coupled with the xanthan gum particle size, appear to be significant to the preparation of a clear and stable composition. The particle sizes of the preferred xanthan gums can also be significant and are such at 100% goes through 60 mesh (250 micron) and at least 95% through 80 mesh (180 microns) and preferably at least 99% through 177 micron and more preferably at least 92% through 200 mesh (80 micron).

The second portion of the clarity, particle stabilization system is the guar gum. Guar gums are generally D-galactose/D-mannose in a molar ratio of 1:2. Guar gums which are useful in this invention are characterized by the same set of parameters and values as the xanthan gum. The guar gum is preferably an alkoxylated guar gum such as ethoxylated or, more preferably propoxylated.

The combination of the xanthan gum with the guar gum brings about composition property enhancement. Clarity of the aqueous phase and stable suspendability of the particles is maintained. This is achieved with approximately the same cost of materials as xanthan gum alone. Additionally, these properties are maintained at a viscosity level approximately 50% higher than using xanthan gum alone. Still further, the composition issues from a container under pressure in a continuous stream rather than squirting. Finally, the composition is smoother flowing rather than having an interrupted flow even when not under any external pressure. Therefore, it appears that the xanthan guar gum mixture provides a different rheology to the composition than xanthan gum alone. Generally, the quantity of xanthan gum is from about 0.4 to about 1.5 wt. %, preferably about 0.6 to about 1.0 wt. % of the composition. The guar gum is from about 0.2 to about 1.5 wt. % preferably about 0.3 to about 0.8 wt. % of the composition. Generally the xanthan gum is present in the composition in greater quantities than the guar gum. However, the opposite can occur as well.

The successful suspending system should also provide the desired viscosity. The composition is to be liquid, pourable and in non-gellular or gellular form. Delivery systems include a hand pumpable container, squeeze container or poured from a container. Generally, the preferred viscosity is from about 3000 to about 15,000 centipoises as measured on a Brookfield RVTD viscometer spindle 4 at 10 rpm and 25°C., more preferably about 5000 to about 10,000 centipoises. Suitable xanthan gums are available from Kelco located in Brussels, Belgium and marketed as Keltrol T or Keltrol TF, preferably Ketrol T. Suitable guar gums are available from Rhone Poulenc, preferably Jaguar HP 105.

The compositions of the invention are prepared by standard techniques taking into account the specific requirements of the composition. The xanthan gum employed is wet into emulsifier, fragrance and preservative or various combination thereof. The guar gum is slurried into a relatively low molecular weight polyethylene glycol of from about 200 to about 600 molecular weight. This combination is then added to the water of the composition using a high shear homogenizer, such as an oscillating knife edge thereby providing clarity and viscoelasticity to the composition. The various surfactants are then added followed by the conditioning bearing particles. The proper viscosity and suspension is achieved. The preferred pH is slightly acidic and more preferably ranges from about 5 to about 7 so as to assist in maintaining proper compatibility with the skin. After optional material(s) are added, the balance of the composition is water.

Aging studies are conducted so as to measure both the clarity (clearness) of the aqueous phase and the stability of the suspended particles. In order to be considered clear, the composition should show less than or equal to about 20 NTU turbidity. The turbidity standard is the water used in the composition (0 NTU). A stable suspension is determined to be no visual settling together of particles at the bottom of the container, and no visual rising of the particles to the top. Additionally, no significant alteration of the particle positions in the container and no observed physical change of the particles such as in shape or color which would indicate an interaction with their environment should occur. The time period assessed is preferably at least three months at 43.3 ° C. No significant modification of foam performance profile of the finished product should be present in that time period. This is an indication that the oily contents of the solid particles are not released.

Below is an examples of the invention demonstrating the uniqueness of the formulation with respect to its components, particularly, the viscoelastic suspending system. This example of the invention is intended to exemplify the invention and not be an undue limitation(s) thereof.

**EXAMPLE 1**

| **Shower Gel Composition With Xanthan and Guar Gum** | |
|---|---|
| **Ingredients** | **% In Formula** |
| D₁ Water | 52.45 |
| Tetra Sodium EDTA-39% | 0.20 |
| FD&C Blue #1, 0.1% Solution | 0.48 |
| D&C Yellow #10, 0.1% Solution | 0.08 |
| Benzophenone-4 | 0.05 |
| Perfume | 0.65 |
| Polyethylene Glycol | 0.30 |
| Dibromo Dicyano Butane- 10% in dipropylene glycol | 0.30 |
| Triclosan | 0.15 |
| Xanthan Gum | 0.80 |
| Polyethylene Glycol 400 | 0.70 |
| DMDM Hydantoin | 0.03 |
| Guar Gum, 2-Hydroxypropyl Ether | 0.40 |
| C12-14 Alcohol EO 2:1 Na Sulphate (25.5%AI) | 37.26 |
| Cocoamidopropyl Betaine NO.3, (30% Al) | 5.66 |
| Citric Acid Solution (50%) | ≤ 0.1 |
| Aqueous Slurry of Microericapsulated Beads | 0.50 |
| **Total** | **100.00** |

After three months at 433°C., the compositions remain visually clear, and no more than 15 NTU turbidity. The particles remain stable as measured visually by no grouping together at the top or bottom as well as change of physical shape. No discoloring of the aqueous phase nor of the particles is observed after three months at 43.3°C. There appears to be no effect on lathering at the end of the aging period of three months. The viscosity of the composition is 7500 centipoise and is readily dispensed in an even flow from a squeeze container. It is more readily smoothly pourable from an open container than a composition of similar viscosity using xanthan guam alone.

## Claims

1. A composition comprising a clear, aqueous cleansing phase having stably dispersed and suspended therein particles bearing a conditioning agent, comprising:
a) a high foaming anionic surfactant,
b) an amphoteric surfactant in quantities from zero wt.% of the composition to a quantity wherein clarity of the aqueous phase is maintained,
c) an effective amount of a viscoelasticity enhancing suspending material mixture comprised of a xanthan gum having an initial transmittance in a 1 wt. % distilled water solution of at least 85% and a guar gum having an initial transmittance in a 1 wt.% distilled water solution of at least 85%,
d) the said particles, insoluble and stably suspended in the aqueous phase, bearing an effective amount of oily water insoluble or essentially water insoluble skin or hair conditioning agents.

2. The composition in accordance with claim 1 wherein (a) is an ethoxylated sulfate or sulfonate.

3. The composition in accordance with claim 1 wherein (b) is a betaine.

4. The composition in accordance with claim 1 where a nonionic surfactant is also present.

5. The composition in accordance with claim 1 wherein the xanthan gum and guargum have a particle size such that 100% of each goes through a 250 micron mesh screen.

6. The composition in accordance with claim 1 wherein the particles bear the oily conditioning agent within or upon the surface of the particle.

7. The composition in accordance with claim 2 wherein the sulfate or sulfonate is 2 wt. % to 20 wt. % of the composition.

8. The composition in accordance with claim 3 wherein the betaine is at least 0.2 wt. % of the composition.

9. The composition in accordance with claim 4 wherein the nonionic surfactant is an alkylated polyglycoside and is from 0.5 wt. % to 10 wt. % of the composition.

10. The composition in accordance with claim 5 wherein the xanthan gum is from 0.4 wt. % to 1.0 wt. % of the composition and the guar gum is from about 0.2 to 1.0 wt. % of the composition..

11. The composition in accordance with claim 6 wherein the particles have an average diameter size of from 800 to 1800 microns.

12. The composition in accordance with claim 1 wherein oily conditioning agents are selected from the group consisting of vitamins, provitamins, emollients, moisturizers, silicones, vegetable oils and mineral oils.

13. The composition in accordance with claim 8 wherein the betaine is cocoamidopropyl betaine.

14. A method of concomitantly cleansing and conditioning the skin or hair which comprises applying the composition of claim 1 to the skin or hair.

15. A process for preparing a composition which comprises mixing the components of claim 1.

## Patentansprüche

1. Zusammensetzung umfassend eine klare, wässrige Reinigungsphase mit darin stabil dispergierten und suspendierten Teilchen, die ein Konditionierungsmittel tragen, umfassend:
a) stark schäumendes anionisches Tensid,
b) amphoteres Tensid in Mengen von 0 Gew.-% der Zusammensetzung bis zu einer Menge, bei der die Klarheit der wässrigen Phase erhalten bleibt,
c) eine wirksame Menge einer viskoelastizitäterhöhenden, suspendierenden Materialmischung aus Xanthan mit einer anfänglichen Durchlässigkeit von mindestens 85 % in einer 1 Gew.-% Lösung in destilliertem Wasser und Guar Gum mit einer anfänglichen Durchlässigkeit von mindestens 85 % in einer 1 Gew.-% Lösung in destilliertem Wasser,
d) diese Teilchen, unlöslich und in der wässrigen Phase stabil suspendiert, die eine wirksame Menge von öligen, wasserunlöslichen oder im Wesentlichen wasserunlöslichen Haut- oder Haarkonditionierungsmitteln tragen.

2. Zusammensetzung nach Anspruch 1, bei der (a) ethoxyliertes Sulfat oder Sulfonat ist.

3. Zusammensetzung nach Anspruch 1, bei der (b) ein Betain ist.

4. Zusammensetzung nach Anspruch 1, bei der ferner nichtionisches Tensid vorhanden ist.

5. Zusammensetzung nach Anspruch 1, bei der Xanthan und Guar Gum eine Teilchengröße aufweisen, so dass 100 % von jedem durch ein 250 µm Mesh Sieb gehen.

6. Zusammensetzung nach Anspruch 1, bei der die Teilchen das ölige Konditionierungsmittel innerhalb oder auf der Oberfläche der Teilchen tragen.

7. Zusammensetzung nach Anspruch 2, bei der das Sulfat oder Sulfonat etwa 2 Gew.-% bis etwa 20 Gew.-% der Zusammensetzung ausmacht.

8. Zusammensetzung nach Anspruch 3, bei der das Betain mindestens 0,2 Gew.-% der Zusammensetzung ausmacht.

9. Zusammensetzung nach Anspruch 4, bei der das nichtionische Tensid ein alkyliertes Polyglucosid ist und 0,5 Gew.-% bis 10 Gew.-% der Zusammensetzung ausmacht.

10. Zusammensetzung nach Anspruch 5, bei der das Xanthan 0,4 Gew.-% bis 1,0 Gew.-% der Zusammensetzung ausmacht und das Guar Gum etwa 0,2 bis 1,0 Gew.-% der Zusammensetzung ausmacht.

11. Zusammensetzung nach Anspruch 6, bei der die Teilchen einen durchschnittlichen Durchmesser von 800 bis 1800 µm aufweisen.

12. Zusammensetzung nach Anspruch 1, bei der die öligen Konditionierungsmittel ausgewählt sind aus der Gruppe bestehend aus Vitaminen, Provitaminen, erweichenden Mitteln, feuchtigkeitsspendenden Mitteln, Silikonen, Pflanzenölen und Mineralölen.

13. Zusammensetzung nach Anspruch 8, bei der das Betain Kokoamidopropylbetain ist.

14. Verfahren zum gleichzeitigen Reinigen und Konditionieren der Haut oder des Haares, bei dem die Zusammensetzung gemäß Anspruch 1 auf die Haut oder das Haar aufgebracht wird.

15. Verfahren zur Herstellung einer Zusammensetzung, das das Mischen der Komponenten gemäß Anspruch 1 umfasst.

## Revendications

1. Composition comprenant une phase aqueuse nettoyante limpide dans laquelle des particules portant un agent de conditionnement sont dispersées et tenues en suspension de manière stable, comprenant :
a) un agent tensio-actif anionique très moussant,
b) un agent tensio-actif amphotère en des quantités allant de zéro % en poids de la composition à une quantité à laquelle la limpidité de la phase aqueuse est maintenue,
c) une quantité efficace d'un mélange de matériaux de suspension accroissant la viscoélasticité, comprenant une gomme xanthane ayant une transmission initiale dans une solution à 1 % en poids dans l'eau distillée d'au moins 85 % et une gomme guar ayant une transmission initiale dans une solution à 1 % en poids dans l'eau distillée d'au moins 85 %,
d) lesdites particules, insolubles et tenues en suspension de manière stable dans la phase aqueuse, portant une quantité efficace d'agents de conditionnement huileux pour la peau ou les cheveux, insolubles dans l'eau ou essentiellement insolubles dans l'eau.

2. Composition selon la revendication 1, dans laquelle (a) est un sulfate ou sulfonate éthoxylé.

3. Composition selon la revendication 1, dans laquelle (b) est une bétaine.

4. Composition selon la revendication 1, qui contient en outre un agent tensio-actif non ionique.

5. Composition selon la revendication 1, dans laquelle la gomme xanthane et la gomme guar ont une taille de particules telle que 100 % de chacune passe à travers un tamis à mailles de 250 micromètres.

6. Composition selon la revendication 1, dans laquelle les particules portent l'agent de conditionnement huileux dans ou sur la surface de la particule.

7. Composition selon la revendication 2, dans laquelle le sulfate ou sulfonate représente environ 2 % en poids à environ 20 % en poids de la composition.

8. Composition selon la revendication 3, dans laquelle la bétaïne représente au moins 0,2 % en poids de la composition.

9. Composition selon la revendication 4, dans laquelle l'agent tensio-actif non ionique est un polyglycoside alkylé et représente 0,5 % en poids à 10 % en poids de la composition.

10. Composition selon la revendication 5, dans laquelle la gomme xanthane représente 0,4 % en poids à 1,0 % en poids de la composition et la gomme guar représente environ 0,2 à 1,0 % en poids de la composition.

11. Composition selon la revendication 6, dans laquelle les particules ont un diamètre moyen de 800 à 1800 micromètres.

12. Composition selon la revendication 1, dans laquelle les agents de conditionnement huileux sont choisis dans la classe formée par les vitamines, les provitamines, les émollients, les hydratants, les silicones, les huiles végétales et les huiles minérales,

13. Composition selon la revendication 8, dans laquelle la bétaïne est la coco-amidopropyl-bétaïne.

14. Procédé pour nettoyer et conditionner simultanément la peau ou les cheveux, qui comprend l'application de la composition de la revendication 1 à la peau ou aux cheveux.

15. Procédé pour préparer une composition, dans lequel on mélange les composants de la revendication 1.
